(19) Europäisches Patentamt
European Patent Office
Office européen des brevets

(11) Publication number: **0 017 503**
**B1**

(12) **EUROPEAN PATENT SPECIFICATION**

(45) Date of publication of patent specification: **29.12.82**

(21) Application number: **80301101.4**

(22) Date of filing: **03.04.80**

(51) Int. Cl.³: **C 07 C 31/18,** C 07 C 31/22, C 07 C 31/20, C 07 C 29/14, C 07 C 29/38, C 08 G 18/32

(54) **Gem-bis (hydroxymethyl) alcohols and mixtures thereof with diols, process for their preparation and a process for the preparation of polyurethanes therefrom.**

(30) Priority: **04.04.79 US 26858**
**04.04.79 US 26859**
**04.10.79 US 81951**
**04.10.79 US 81953**

(43) Date of publication of application:
**15.10.80 Bulletin 80/21**

(45) Publication of the grant of the patent:
**29.12.82 Bulletin 82/52**

(84) Designated Contracting States:
**BE CH DE FR GB IT LI NL SE**

(73) Proprietor: **HENKEL CORPORATION**
**4620 West 77th Street**
**Minneapolis Minnesota 55435 (US)**

(72) Inventor: **Rogier, Edgar R.**
**16400 Hidden Valley**
**Minnetonka Minnesota 55343 (US)**

(74) Representative: **Watkins, Arnold Jack et al,**
**European Patent Attorney Frank B. Dehn & Co.**
**Imperial House 15-19 Kingsway**
**London WC2B 6UZ (GB)**

(56) References cited:
**EP - A - 0 000 912**
**FR - A - 1 335 323**
**FR - A - 1 377 140**
**FR - A - 1 447 188**
**FR - A - 2 009 683**
**FR - A - 2 069 018**
**FR - A - 2 301 505**
**FR - A - 2 385 751**
**GB - A - 808 878**

(56) References cited:
**THE JOURNAL OF THE AMERICAN OIL CHEMISTS SOCIETY, vol. 54, no. 11 November 1977, pages 882A—885A Minneapolis, U.S.A. W. R. MILLER et al.: "Geminal hydroxymethyl compounds from 9(10) formyl steroc Acid".**
**CHEMIKER ZEITUNG, vol. 101, 1977, no. 7/8, pages 343—350 Heidelberg, DE. B. FELL et al.: "Isomerisierungsfreie Hydroformylierung mit Rhodiumcarbonyl/tert. Phosphin-Komplexkatalysatoren, II. Die Hydroformylierung von Allylalkoholen".**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 017 503 B1**

Courier Press, Leamington Spa, England.

# Gem-bis (hydroxymethyl) alcohols and mixtures thereof with diols, process for their preparation and a process for the preparation of polyurethanes therefrom

This invention relates to products and processes useful in the manufacture of synthetic resins.

Hydroformylation is basically defined as the addition of a formyl group through the reaction of an unsaturated compound with carbon monoxide and hydrogen. The basic technology for the manufacture of hydroformylated products and consequently their derivatives is amply set out hereinafter. Among the difficulties which must be met in the manufacture of hydroformylated products is the consideration that hydrogen gas, an explosive, and carbon monoxide, a hazardous material, are utilized in the process. Hydroformylation processes are also dependent on expensive metallic catalysts such as carbonyls which have high toxicity and high cost. The conditions for running a hydroformylation reaction also involve the use of substantial temperature and pressure thus necessitating costly equipment which must be maintained.

Thus due to the various factors and considerations which go into the manufacture of hydroformylated products and their derivatives it is essential that the reactions individually and cumulatively give high purity of the desired end product and high yield thereby avoiding excessive handling of hazardous materials while minimizing the high capital cost and maintenance of such production facilities.

In the past several attempts have been made to prepare hydroformylated products or similar materials such as is described in United States Patent 2,437,600 to Gresham et al issued March 9, 1948.

A brief discussion of this and other publications relating to the technique of hydroformylation may be found in European Patent Application Publication No. 17503A of Henkel Corporation.

Compounds obtained through hydroformylation technology useful as plasticizers are discussed in a Frankel et al article entitled "Acyl Esters from Oxo-Derived Hydroxymethylstearates as Plasticizers for Polyvinyl Chloride" printed in the J. Am. Oil Chemists' Soc. 52, p. 498—504 (1975).

Useful diols for resin purposes are described in United States Patent 2,933,477 issued April 19, 1960 to Hostettler. Nonadecanediols are described as being utilized in urethane formulations in United States Patent 3,243,414 to DeWitt et al issued March 29, 1966. The production of triols which are particularly useful in resins due to the close positioning of the hydroxyl groups is reported in "Improved Synthesis of 1,1,1-trimethylolalkanes from Hexanol and Nonanal", J. Am. Oil Chemists' Soc. 45, p. 517 (July 1968) by Moore and Pryde.

Frankel and Pryde in an article entitled "Catalytic hydroformylation and hydrocarboxylation of unsaturated fatty compounds" J. Am. Oil Chemists' Soc. *54* (1977) 873A have discussed the use of the Tollen's reaction in the treatment of formyl stearic acids to obtain gem-bis-hydroxymethyl stearic acids. In such gem-bis-hydroxymethyl stearic acids however, the carboxyl functionality has the potential to form polymers and/or internal esters through reaction with the hydroxyl groups.

Szabo et al. in French Patent No. 1447188 (Unilever N.V.) disclose a gem-bis-hydroxymethyl alcohol having an aliphatic carbon chain skeleton. This triol, 4,4-bis-(hydroxymethyl)-tetradecan-1-ol, was obtained by reduction of the ester of the corresponding tricarboxylic acid and was found to be solid at ambient temperature having a melting point of 71°C.

Each of the foregoing to the extent that it is applicable to the present invention is herein incorporated by reference.

One object of the present invention is to describe the preparation of high molecular weight polyhydric alcohols and their useful urethane reaction products formed by the condensation of the polyhydric alcohol with a polyisocyanate. Of course several other uses of the technology embodied in this patent are readily apparent.

Throughout the specification and claims of the present invention percentages and ratios are by weight and temperatures are in degrees of Celsius unless otherwise indicated.

According to one aspect of the present invention we therefore provide a *gem*-bis (hydroxymethyl) alcohol of formula I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[wherein m, n, p, q, r, s and t are integers the sum of which is z which has a value of from 12 to 20; n, q and s each has a value of 0 or 1 with the sum of n, q and s, which is y, having a value of from 1 to 3; m, p and r, which may be the same or different, each has a value of from 0 to 16; and t has a value of from 3 to 19].

According to a second aspect of the present invention we provide a mixture of a *gem*-bis (hydroxymethyl) alcohol of formula I as defined above and a diol of formula IIA

$$H{+}CH_2{+}_{n'}\!-\!\overset{\displaystyle CH_2OH}{\overset{\vdots}{C}H}{+}CH_2{+}_{k'}CH_2OH \qquad (IIA)$$

[wherein h′ and k′ are integers; k′ is greater than or equal to 3; h′ is greater than or equal to 1; and the sum of h′ and k′ is from 12 to 20].

*Gem*-bis (hydroxymethyl) alcohols of formula I which, by virtue of their properties, are preferred according to the present invention are those in which at least one of the following conditions is satisfied:

i) z is from 14 to 18;
ii) y is 1 and z is 16;
iii) y is 2 and z is 16;
iv) y is 3 and z is 16;
v) q is 1 and n and s are zero;
vi) m and t, which may be the same or different, each has a value greater than or equal to 4;
vii) n, p, r and s are zero and q is 1;
viii) t is 7 or 8, s is 1, r is 7 or 8, z is 16 and m, n, p and q are zero;
ix) t is 7 or 8, s is 1, r is 1, 2 or 3, q is 1, p is 4 or 5, z is 16 and m and n are zero; and
x) t is 7 or 8, s is 1, r is 1, 2 or 3, q is 1, p is 1, 2 or 3, n is 1, m is 1 or 2 and z is 16.

Mixtures of diols of formula IIA and *gem*-bis (hydroxymethyl) alcohols of formula I which, by virtue of their properties, are preferred according to the present invention are those in which at least one of the following conditions is satisfied:

$\alpha$) the sum of h′ and k′ is from 14 to 18;
$\beta$) the sum of h′ and k′ is equal to z, h′ is greater than or equal to t and k′ is greater than or equal to (m+1);
$\gamma$) m, t and h′, which may be the same or different, each has a value greater than or equal to 4;
$\delta$) n, p, q, r and s are zero and q is 1;
$\varepsilon$) the diol is 9 (10) hydroxymethyl octadecanol;
$\zeta$) the *gem*-bis (hydroxymethyl) alcohol is 9,9,(10,10)bis(hydroxymethyl) octadecanol;
$\eta$) the *gem*-bis (hydroxymethyl) alcohol is 9,9(10,10); 12,12(13,13)di[bis(hydroxymethyl)] octadecanol;
$\theta$) the *gem*-bis (hydroxymethyl) alcohol is 9,9(10,10); 12,12(13,13); 15,15(16,16)tri[bis-(hydroxymethyl)]octadecanol; and
$\iota$) the weight ratio of gem alcohol to diol is from about 1:2 to about 100:1.

Formyl alcohols of formula III (as defined below) are useful intermediates in the production of diols or *gem*-bis (hydroxymethyl) alcohols according to the present invention. Although in the processes described below involving the use of such formyl alcohols as reagents it is preferable that the formyl alcohols be produced by a process as described below, this need not be the case.

The formyl alcohols of formula III

$$CH_3-(CH_2)_m-\left[\begin{array}{c}CH\\|\\CHO\end{array}\right]_n-(CH_2)_p-\left[\begin{array}{c}CH\\|\\CHO\end{array}\right]_q-(CH_2)_r-\left[\begin{array}{c}CH\\|\\CHO\end{array}\right]_s-(CH_2)_t-CH_2-OH \qquad (III)$$

[wherein m, n, p, q, r, s and t are integers the sum of which is z which has a value of from 12 to 20; n, q and s each has a value of 0 or 1 with the sum of n, q and s, which is y, having a value of from 1 to 3; m, p and r, which may be the same or different, each has a value of from 0 to 16; and t has a value of from 3 to 19] which may be used as intermediates in the preparation of the compounds of the invention may be prepared by a process in which an unsaturated alcohol of formula IV

$$H-[CH_2]_a-[CH{=}CH]_b-[CH_2]_c-[CH{=}CH]_d-[CH_2]_e-[CH{=}CH]_f-[CH_2]_g-CH_2-OH \qquad (IV)$$

[wherein a, b, c, d, e, f and g are integers; b, d and f respectively have the values n, q and s; a, which is non-zero, has a value of m or (m+1); c has a value of from p to (p+2); e has a value of from r to (r+2); g has a value of t or (t+1); the sum of a, c, e and g is x; (x+2y) is equal to (z+1); and m, n, p, q, r, s, t, y and z are as hereinbefore defined] is subjected to hydroformylation in the presence of a rhodium-containing catalyst.

In the process described above it is preferred if at least one of the following conditions is satisfied:

a) n, p, r and s are all zero, q is 1 and the sum of m and t is from 11 to 19;
b) z has a value of from 14 to 18;
c) m and t, which may be the same or different, each has a value greater than or equal to 4;
d) n and s are zero and q is 1;
e) the unsaturated alcohol of formula II (as defined above) is oleyl alcohol;
f) the unsaturated alcohol of formula II (as defined above) is linoleyl alcohol; and
g) the unsaturated alcohol of formula II (as defined above) is linolenyl alcohol.

It is particularly preferred that at least one of the conditions (a), (b), (c), (d) and especially (e) given above is satisfied.

According to a further aspect of the present invention we therefore provide a process for the preparation of a *gem*-bis (hydroxymethyl) alcohol of formula I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[wherein m, n, p, q, r, s and t are as defined above, advantageously having the preferred values stated above] wherein a formyl alcohol of formula II (as defined above) is subjected to a Tollen's reaction, preferably in the presence of an inert atmosphere (such as, for example, nitrogen) and a strong base.

The diols of formula II

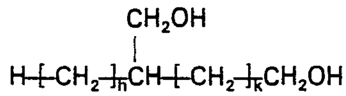

(II)

[wherein h and k are integers; k is greater than or equal to t (wherein t is as hereinafter defined); h is greater than or equal to m+1 (wherein m is as hereinafter defined); and the sum of h and k is equal to z (wherein z is as hereinafter defined)] which are components of the mixtures of the invention may be prepared by the reduction by hydrogenation of a formyl alcohol of formula I (as defined above and wherein m, n, p, q, r, s, t are integers as defined above the sum of which is z, these integers advantageously having the preferred values stated above).

*Gem*-bis (hydroxymethyl) alcohols of formula I (as defined above) can also be prepared by the reduction of hydroxymethyl formyl alcohols of formula V (as defined below).

According to a further feature of the present invention we therefore provide a process for the preparation of a *gem*-bis (hydroxymethyl) alcohol of formula I as defined above wherein a hydroxymethyl formyl alcohol of formula V

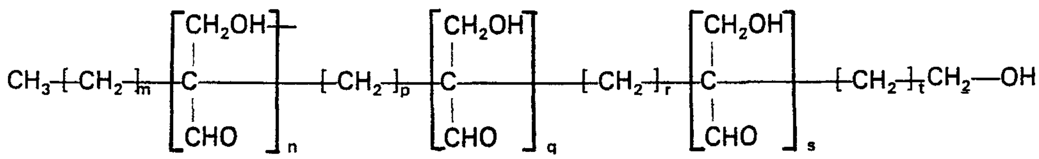

(V)

[wherein m, n, p, q, r, s and t are as defined above, advantageously having the preferred values stated above] produced by the subjection of a formyl alcohol of formula III (as defined above) to an aldol condensation reaction, preferably in the presence of a weak base such as, for example, triethylamine, is reduced to produce the said *gem*-bis (hydroxymethyl) alcohol.

The gem-bis (hydroxymethyl) alcohols and mixtures thereof with diols according to the present invention can be advantageously employed in the preparation of polyurethanes.

Thus, according to the present invention we also provide a process for the preparation of a polyurethane from a *gem*-bis (hydroxymethyl) alcohol of formula I (as defined above) or from a mixture of the said *gem*-bis (hydroxymethyl) alcohol and a diol of formula II or IIA (as defined above) by reaction with an organic polyisocyanate.

The preparation of the intermediate formyl alcohols of formula III for use in processes of the present invention involves hydroformylation which is the process for the production of aldehydes from olefinically unsaturated compounds by reaction with carbon monoxide and hydrogen in the presence of a catalyst. The aldehydes produced generally correspond to the compounds obtained by the addition of a hydrogen and a formyl group to an olefinicably unsaturated group in the starting material thus saturating the olefinic bond.

Most preferably the starting raw material is oleyl alcohol although linoleyl or linolenyl alcohol may be employed. It is of course noted that any number of synthetic unsaturated alcohols may also be employed in the present invention. However, for most purposes the naturally occurring alcohols derived from plant sources are presently most convenient and inexpensive.

The unsaturated alcohol is reacted with hydrogen gas and carbon monoxide in the presence of a rhodium catalyst as later described to form the corresponding formyl alcohol having the formula

$$CH_3(CH_2)_m[CH(CHO)]_n(CH_2)_p[CH(CHO)]_q(CH_2)_r[CH(CHO)]_s(CH_2)_tCH_2OH$$

wherein the various subscript numbers are as previously described.

The addition of hydrogen and carbon monoxide is accomplished in practice by conveniently adding stoichiometric amounts of the hydrogen and carbon monoxide to give the formyl alcohol. To assure completeness of the reaction the amounts of hydrogen and carbon monoxide may be each maintained at from about 1.5:0.5 to about 0.5:1.5 molar ratio to one another. It is noted that the ratio is not critical as long as the pressure is maintained in the reaction vessel by the component gases and that the amount of hydrogen is not so great as to substantially reduce the unsaturated starting material.

The rhodium catalyst as later described is necessary in the hydroformylation reaction in that it has

been found that the use of the more conventional cobalt catalyst results in a substantial amount of cross-linking gelation. It is believed that the gelation is due to the coproduction of polyhemiacetals and polyacetals in competition with the production of the hydroformylated alcohol. It was at first believed by the author that it would be necessary, even with a rhodium catalyst, to employ the ester of the unsaturated alcohol e.g. oleyl acetate to avoid the unwanted by-products. Of course the ester is more expensive and eventually is converted to the alcohol in any event.

Higher yields of product are obtained through the use of the rhodium catalysts than if a cobalt catalyst is employed. It has also been observed that a much higher degree of isomerization of the double bond occurs with a cobalt catalyst than with a rhodium catalyst.

The temperature during the hydroformylation is conveniently from about 90 degrees to about 170 degrees C, preferably from about 110 degrees C to about 130 degrees C. Above the higher temperatures listed above increased amounts of unwanted by-products are formed in the reaction mixture. The pressure conditions are such that the pressure in sealed system is maintained at from about 20 to about 500 atmospheres, preferably from about 30 to about 100 atmospheres absolute during the hydroformylation.

The preferred end product obtained from conducting the foregoing process is 9(10) formyl octadecanol when the starting material is oleyl alcohol. The positioning of the 9(10) indicates that the product obtained is a mixture of the 9 and 10 isomer with respect to the formyl group. One additional reason for using a rhodium catalyst is that if a cobalt catalyst were employed a considerable amount of terminal aldehyde would be formed due to bond migration prior to the addition of the formyl group. When the terminal aldehyde group is formed the resultant alcohol obtained by carrying out the remainder of the herein described process is unsuitable for many of the purposes that the geminal alcohols may be utilized for.

It should also be appreciated that if 9,12-linoleyl alcohol is the starting material then the formyl alcohol so formed will be a 9(10), 12(13) diformyloctadecanol. That is, the end product obtained here will actually be a mixture of the 9—12, 9—13, 10—12, 10—13 diformyl alcohols. Similarly without discussing all the particular isomers present when 9,12,15-linolenyl alcohol is employed the product so obtained will be a mixture of the 9(10), 12(13), 15(16) triformyloctadecanol isomers.

It is particularly desirable that the expensive rhodium catalyst is recovered. This may be conveniently done by distillation of the formyl alcohol leaving the rhodium in the residue. What is particularly surprising is that the rhodium can be recovered from the distillate in that the art would predict that when hydroformylating an unsaturated alcohol that the products obtained would include considerable quantities of polyhemiacetals and polyacetals as a portion or all of the reaction product and that these products would not be recoverable by distillation. Thus not only is the desired end product achieved in a high degree of purity and yield through the use of the rhodium catalyst but the rhodium catalyst is recoverable in extremely high quantities from the reaction mixture.

It should also be emphasized that if the polyhemiacetals and polyketals were formed in the reaction mixture that it is very likely that the reaction components would undergo a great change in viscosity to the point of forming a semi-solid product due to the extensive cross-linking of the acetal and ketal linkages. Thus a substantial reason exists for avoiding the polyhemiacetal and polyacetal formation through the use of a rhodium catalyst.

It may be stated that the polyacetal and polyhemiacetal formation might be prevented by the utilization of the corresponding unsaturated acid or its ester in place of the unsaturated alcohol.

However, this substitution which eventually involves the acid ester is undesirable in that an aqueous neutralization step is required which forms a soap as a by-product. The soap so formed then emulsifies the reaction products and the water present to make separation extremely difficult thus diminishing recovery of both the alcohol and the expensive catalyst. Thus the present invention is highly selective to both unsaturated alcohol and the particular rhodium catalyst so employed.

Any convenient source of rhodium may be employed as in the present reaction mixture the rhodium catalyst is actually converted through the presence of the hydrogen and carbon monoxide into its active form which is a rhodium carbonyl hydride. Conveniently the source of rhodium for use in the rhodium catalyst may be rhodium metal, rhodium oxide, and various other rhodium salts such as rhodium chloride, rhodium dicarbonyl chloride dimer, rhodium nitrate, rhodium trichloride and other similar materials.

The rhodium catalyst in the present hydroformylation reaction is preferably present with a ligand such as a trisubstituted phosphine or trisubstituted phosphite. The term trisubstituted includes both alkyl and aryl compounds and the substituted compounds of the alkyl and aryl compounds. A particularly valuable ligand for the rhodium carbonyl hydride is triphenylphosphite or triphenylphosphine in that both compounds are particularly useful in minimizing migration of the double bond thereby avoiding a large number of isomers with respect to the formyl group including the undesired terminal formyl compound as previously discussed. In general triaryl phosphines or triarylphosphites may be used for this purpose in the formation of the rhodium carbonyl hydride ligand. In addition, the foregoing materials are extremely valuable in minimizing the undesired reaction of saturation of the double bond or the reduction formyl group. This frequently occurs in the absence of

such ligands because the rhodium catalyst functions excellently as a hydrogenation catalyst. That is the ligand tends to eliminate such side reactions.

In general any one of several other additional ligands may be used with the rhodium catalyst. Such additional ligands are discussed in the "Selective Hydroformylation of Unsaturated Fatty Acid Esters" by Frankel in the Annals N.Y. Academy of Sciences 214:79 (1973).

The various ligands are conveniently employed in mole ratio to the rhodium metal content of the catalyst of from about 2 to 50 preferably from about 3 to 20. The rhodium catalyst based upon its metal content is conveniently employed in catalytic amounts preferably from about 22 ppm to about 10,000 ppm, most preferably from about 50 ppm to about 500 ppm by weight of the unsaturated alcohol.

The various formyl alcohols are useful as previously stated in preparing the highly desired *gem*-bis (hydroxymethyl) alcohols. The alcohols may be formed from the foregoing formyl alcohols via a Tollen's reaction (aldol condensation followed by a crossed-Cannizzaro reaction).

Schematically the Tollen's reaction is as described below.

$$RCH(CHO)R+HCHO \xrightarrow{\text{weak base}} RC(CHO)(CH_2OH){-}R \qquad (A)$$

$$(A) \quad +HCHO+MOH \rightarrow RC(CH_2OH)_2R+HCO_2M$$

wherein the above formula R indicates an organic moiety, compound (A) is a hydroxymethyl aldehyde and MOH is a strong base.

The Tollens' reaction is thus carried out by reacting one mole of a monoformylated alcohol with two moles of formaldehyde in an inert atmosphere such as nitrogen. Where the formyl alcohol contains more than one formyl group, two moles of formaldehydes are required for each formyl group present. Thus if the reactant is formyloctadecanol then two moles of formaldehyde are required for conversion to the *gem*-bis(hydroxymethyl) alcohol whereas if linoleyl alcohol is utilized in the first instance to give a diformyloctadecanol then four moles of formaldehyde are required to obtain the digeminaloctadecanol. Conveniently an excess of up to 1.5 preferably up to 1.2 times the amount of formaldehyde actually required to form the corresponding *gem*-bis (hydroxymethyl) alcohol is employed in the present invention. A convenient manner of adding the formaldehyde in the Tollens' reaction is by using a methanol solution of formaldehyde.

The Tollens' reaction utilizes a strong base as both a reactant and a catalyst. Such strong bases include sodium, potassium or calcium hydroxide. Other strong bases such as carbonates or other hydroxides may be used as well. The strong base is conveniently employed on an equivalent basis per formyl group to convert the formyl group to the hydroxy methyl group. The amount of base required in the Tollens' reaction is at least an equivalent of that required preferably up to 1.5, most preferably up to 1.2 equivalents. The Tollens' reaction is conducted at a temperature of from about 0 degrees C to about 100 degrees C, preferably from about 20 degrees C to about 70 degrees C.

The crude *gem*-bis(hydroxymethyl) alcohol so formed is washed with water to remove any excess caustic and salts formed and then obtained in a relatively pure state by vacuum drying.

In obtaining the *gem*-bis(hydroxymethyl) alcohol of the present invention the crossed-Cannizzaro reaction predominates over the rate of reaction for the simple Cannizzaro reaction. The Cannizzaro reaction which is promoted by base, water, and heat is the process by which an aldehyde reacts with itself to form the corresponding alcohol and formate salt. That is, in the present invention the formyl group on the formyl alcohol reacts faster with formaldehyde to give the alcohol than does the formaldehyde react with itself.

It is also surprising that the formation of hemiacetal which may be acid or base catalyzed does not occur upon the addition of base to the formyl alcohol while forming the intermediate hydroxymethyl formyl alcohol. Thus two potential side reactions, the Cannizzaro and the hemiacetal formation (and thereafter the acetal) which might be expected given the reactants and the processing conditions involved do not in fact occur and the useful alcohol is obtained in substantial quantities.

It has been found, however, in the present invention that the more complicated crossed-Cannizzaro surprisingly predominates in rate and amount of product (*gem*-bis(hydroxymethyl) alcohol) produced despite the steric hindrance of the larger formyl alcohol molecule even under conditions which are known to promote the simple Cannizzaro reaction.

An alternative method of accomplishing the formation of the geminal alcohol is to use only about one-half the equivalent amount of the formaldehyde required in the Tollens' reaction thereby forming the corresponding hydroxymethyl formyl alcohol via the aldol condensation. That is, the hydroxymethyl group is attached to the carbon in the alpha position to the formyl group. Where a polyformyl alcohol is the intermediate product the formaldehyde is halved from that utilized in the Tollens' reaction to give the corresponding polyhydroxymethyl polyformyl alcohol.

This variation of forming the geminal alcohol eliminates the need for the strong base required in the Tollens' reaction and utilizes instead only catalytic amounts of base which may be either a weak or strong base. A preferred weak base is triethylamine. Even here some care must be taken as it is possible

even when using a weak base to obtain compound (A) as the Cannizzaro reaction may complete with the aldol condensation.

The hydroxymethyl formyl alcohol so formed by this alternative route is then reduced to the alcohol conveniently by using hydrogen gas and a suitable hydrogenation catalyst such as copper chromite, or nickel, via conventional hydrogenation practice or by lithium aluminum hydride production. A significant advantage to the alternative route is the absence of large amounts of salt and solvents needed in the Tollens' reaction route.

A distinct advantage in the geminal alcohol of the present invention is that it is a liquid at room temperature and further has no tertiary hydrogens which are a weak point for chemical attack on the molecule.

A mixture of the diol and the geminal alcohol may be obtained by reacting the unsaturated alcohol to obtain the corresponding formyl alcohol. Thereafter the formyl alcohol is split into two streams, the first of which is processed as previously discussed to give the geminal alcohol while the second stream is reduced by hydrogenation to give the diol. The hydrogenation is generally carried out as discussed in the alternative route for preparing the geminal alcohol. The diol and the geminal alcohol are then recombined in the desired proportions which are preferably in a weight ratio of from about 2:1 to about 1:100 more preferably from about 1:1 to about 1:75. The preferred value for the sum of h and k is from 14 to 18. The liquid nature of the geminal alcohol aids in solubilizing the normally solid diol thus giving a product which is easy to compound.

The products of the present invention are useful in preparing polyurethanes. Among the products which may be obtained from the alcohols are rigid foams and thermosetting elastomers. Additional uses of the *gem*-bis(hydroxymethyl) alcohols of the present invention include scalants, polyvinyl alcohol plasticizers, and poly(oxy)alkylene adducts such as ethylene and propylene oxide adducts for polyurethanes or detergents. The poly(oxy)alkylenes and their halogenated derivatives (especially chlorinated) are particularly useful in polyurethanes. The alcohols formed in the present invention may be reacted with an anionic species to give surfactant products such as the sulfated reaction products of the alcohol. Additional surfactants may be formed by first adducting the alcohol with a poly(oxy)alkylene such as ethylene or propylene oxide and then adding the sulfate group. The present invention also contemplates the caprolactone adducts of the alcohols.

Further uses of the present invention include microcellular foams, the reaction of the alcohol with materials such as acrylic or methacrylic acids to give radiation curable coatings. The alcohols of the present invention may also be used as lubricants or oil substitutes. A further use of the present alcohol compounds are their formation into glycidyl ethers and their subsequent use as a new type of epoxy coating. A further use of the geminal alcohol is in cosmetic preparations particularly as an emollient.

A primary aspect of the present invention, however, is the use of the alcohols so formed as reaction products with isocyanates to form polyurethanes. To form the urethane reaction product of the alcohol a reaction is conducted which requires the presence of an organic polyisocyanate compound.

Suitable polyisocyanates include ethylene diisocyanate, trimethylene diisocyanate, hexamethylene diisocyanate, propylene-1, 3-diisocyanate, ethylidene diisocyanate, cyclopentylene-1, 3-diisocyanate, the 1,2-, 1,3- and 1,4-cyclohexylene diisocyanates, the 1,3- and 1,4-phenylene diisocyanates, polymethylene polyphenylene-isocyanates, the 2,4- and 2,6-toluene diisocyanates, the 1,3- and 1,4-xylylene diisocyanates, bis(4-isocyanatophenyl) methane, 4,4'-diphenyl-propane diisocyanates, bis(2-isocyanatoethyl) carbonate, 1,8-diisocyanato-p-methane, 1-methyl-2, 4-diisocyanato-cyclohexane, the chlorophenylene diisocyanates, naphthalene-1, 5-diisocyanate triphenylmethane-4,4', triisocyanate, isopropylbenzene-alpha-4-diisocyanate, 5,6-bicyclo[2.2.1]hept-2-ene diisocyanate, 5,6-disocyanatobutylbicyclo[2.2.1]hept-2-ene and similar polyisocyanates.

Of particular interest in the present invention are trimethylene hexamethyl diisocyanate available from VEBA, heptadecyl (C17) diisocyanate, DDI 1410 an aliphatic C-36 diisocyanate available from the Henkel Corporation of Minneapolis, Minnesota. (Generally diisocyanates having from 12 to 40 carbons in the aliphatic radical may be used in the present invention), toluene diisocyanate available from Allied Chemical, isophorone diisocyanate available from VEBA and Desmodur N an aliphatic triisocyanate available from Mobay, Desmodur N is more particularly defined the tri-isocyanate adduct of 3 moles of hexamethylene diisocyanate and water having an isocyanate equivalent weight as later defined of 191 grams. Other adducts or prepolymers of the polyisocyanate include Desmodur L and Mondur CB which are the adducts of toluene diisocyanate. The foregoing materials have an isocyanate equivalent weight of approximately 250 grams.

The amount of the polyisocyanate utilized in forming the urethane compositions of the present invention is expressed on a percentage equivalent weight basis with respect to the hydroxyl functionality of the alcohol. Desirably each hydroxyl functional group on the alcohol will react on a 1:1 stoichiometric basis with the isocyanate functionality on the polyisocyanate compound. It is quite feasible, however, to form the urethane linkage using from about 80% to 120% preferably from about 95% to 105% on a hydroxyl-isocyanate equivalent basis of the polyisocyanate to form the urethane product.

To determine the amount of the polyisocyanate required for a given saturated polyol the hydroxyl or isocyanate equivalent weight of the respective polyol or polyisocyanate is determined as that weight

in grams of the material which contains 1 gram equivalent weight of the respective functional group. More particularly to determine the number of equivalents in a given saturated polyol the hydroxyl value is first determined by known methods and reported in milligrams of potassium hydroxide. The calculation to determine the hydroxyl equivalents is then given by the following equation:

$$\text{OH equivalent weight} = \frac{56{,}100}{\text{OH value}}$$

where 56,100 is the milligram equivalent weight of potassium hydroxide.

Alternatively if the weight percentage of the hydroxyl groups in the saturated polyol is known the hydroxyl equivalent is determined as follows:

$$\text{OH equivalent weight} = \frac{17 \times 100}{\text{wt\% OH}}$$

where 17 is the equivalent weight of the hydroxyl radical and the weight percent OH is the percentage of the saturated polyol which is hydroxyl groups.

In similar fashion the isocyanate equivalent may be determined if the weight percent of the isocyanate functional groups in the polyisocyanate is known. This equation is given below where 42 is the molecular weight of an isocyanate functional group and the weight percent NCO is that portion of polyisocyanate made up of isocyanate functional groups.

$$\text{isocyanate equivalent weight} = \frac{42 \times 100}{\text{wt\% NCO}}$$

To form the urethane reaction product the alcohol of the present invention and the organic polyisocyanate are merely mixed together in the proper proportions. When utilized as a coating the compounds are then quickly spread with a knife blade brush or spray over the surface of the article to be coated. Where molded articles are desired various techniques such as reaction injection moulding may be used. Specific techniques for forming urethane reaction products are hereinafter described in the examples.

If desired various urethane catalysts may be employed to promote the reaction. Examples of such urethane catalysts include triethylene diamine, morpholine, N-ethyl-morpholine, dimethyl piperazine, triethylamine, N,N,N',N'-tetramethylbutane-1, 3-diamine, dibutyl-tin dilaurate, stannous octoate, stannous laurate, dioctyl-tin diacetate, lead octoate, stannous oleate, stannous tallate, dibutyl-tin oxide, and hexabutyl-di-tin as well as other art recognized urethane catalysts. Typical levels of the urethane catalyst are from about 0.001% to about 5% by weight of the urethane linking components.

An additional polyol may be included with the alcohols of the present invention. Such polyols may be an alkyl or cycloalkyl polyol, an ester linked polyol, an ether linked polyol, an ether and ester linked polyol or hydroxy functional acrylic copolymers.

Specific examples of alkyl and cycloalkyl polyols include 2,5-hexanediol available from Aldrich Chemical, 1,6-hexanediol, available from Celanese Chemical, ethylene glycol available from Baker, Dimerol a 36 carbon essentially linear diol available from General Mills Chemicals, Inc., glycerol, 1,2-6-hexanetriol available from Union Carbide, pentaerythritol, and 1,4-cyclohexane diol. Additional examples of such polyols include Polybd R-45HT a Butadiene diol having an approximate molecular weight of 2800 available from Arco and Trimethylol propane available from Celanese.

The ester linked saturated diols of the present invention are more particularly described as polyols where the predominate linkage (functional group other than the hydroxyl) are ester radicals. The ester linked saturated polyols are structurally represented as

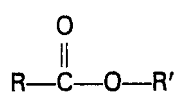

where R and R' are organic residues which contain at least two hydroxyl radicals and at least one ester link.

Examples of ester linked saturated polyols include Niax PCP0200 and PCP0240 both available from Union Carbide and having respective molecular weights of approximately 530 and 2000. Both of the foregoing compounds are diols. Niax PCP0300 also available from Union Carbide is a Caprolactone-ester triol having approximate molecular weight of 540. Niax PCP0310 also available from Union Carbide is a Caprolactone-ester triol having a molecular weight of approximately 900.

The ether linked saturated polyols of the present invention include compounds such as diethylene

glycol and triethylene glycol both available from Fisher. Further ether linked saturated polyols useful in the present invention include the Polymeg Q0650, Q0100, and Q0200 all of which are ether diols available from Quaker having a respective molecular weight of approximately 650, 1000 and 2000. Pluracol P1010 having an approximate molecular weight of 1050 available from Wyandotte is an example of a polypropylene oxide ether linked diol useful in the present invention. Similar Wyandotte products useful as saturated polyols in the present invention include Pluracol TP440 and 150 which are propylene oxide ether linked triols having respective molecular weights of approximately 425 and 1560. In similar fashion Pluracol GP3030 is another saturated polyol suitable for the present invention available from Wyandotte. The foregoing material is a glycerine polypropylene ether linked triol having an approximate molecular weight of 2900.

Additional Pluracols useful in the present invention include Pluracol PEP450 which is pentaerythritol polypropylene oxide ether linked tetrol having a molecular weight of 405 and Pluracol 493 an ether linked tetrol having a molecular weight of approximately 3630.

Ester and ether linked saturated polyols suitable in the present invention are described structurally as

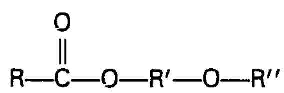

where R, R′ and R″ are organic residues containing at least two hydroxyl radicals and at least one ester and one ether linkage.

The following exemplify the present invention.

Example I

The manufacture of the formyloctadecanol for use in a process according to the present invention may be accomplished by charging a liter Magne Drive, 316 SS autoclave with 606 grams (2.26 moles) of oleyl alcohol, 3.01 grams of 5% rhodium on alumina and 3 grams (9.68 moles) of triphenylphosphite.

The autoclave is sealed and pressurized to 10 atmospheres with nitrogen under stirring and then vented to atmospheric pressure. The nitrogen purge is repeated twice more to ensure removal of any oxygen present in the autoclave.

The autoclave is then pressurized a third time with premixed carbon monoxide and hydrogen gas in a 1 to 1 molar ratio to 68 atmospheres at which point heating is started. Stirring is manually controlled at 1250 rps and the uptake of the mixture of the gases starts at about 100 degrees C.

The reaction conditions are then maintained at a temperature of 130 degrees C and the gas pressure at 70 to 75 atmospheres.

The reaction is substantially complete after 4.6 hours and is determined by the cessation of the gas uptake. The confirmation of the completeness of the reaction is obtained by sampling the mixture and determining through gas chromatograph analysis that there is less than 1% of the starting alcohol in the mixture.

The reaction mixture is then cooled to 75 degrees C, vented to atmospheric pressure and purged twice with nitrogen. The contents of the autoclave are then discharged at 75 degrees C under nitrogen pressure through a pressure filter. The yield of the formyloctadecanol is greater than 90%. Atomic absorption analysis of the filtered product showed 244 ppm of rhodium.

The reaction may be modified by using triphenylphosphine in place of the triphenylphosphite. Alternatively the oleyl alcohol may be substituted for by linoleyl or linolenyl alcohol. The reaction temperature may also be lowered to 90 degrees C at which point the reaction takes a substantially longer period of time to proceed. As a second alternative the reaction temperature can be raised to about 170 degrees C and the reaction time considerably lowered. However, some decomposition of the end product may occur above the 170 degree figure so it should not be exceeded.

In similar fashion the mixture of carbon monoxide and hydrogen may be varied as previously described herein and may also be varied between about 20 and 500 atmospheres of pressure. The lower end of the pressure range of course slows the reaction rate down while the higher pressure condition increases the reaction but also increases the probability that some of the starting alcohol will be saturated by the hydrogen.

Example II

5.26 moles (1570 grams) of the formyloctadecanol obtained from Example I is charged into a 5 liter glass round bottom reaction flask equipped with a heat exchanger coil, thermocouple, stirrer addition inlet, reflux condensor, and combination glass electrode. A further reaction charge of 695 grams (12.87 moles) of a 55.6% formaldehyde in methanol solution is added under a nitrogen blanket. A 40% solution of sodium hydroxide is made by dissolving 245.7 grams (5.95 moles) of sodium hydroxide in 268 grams of water under a nitrogen blanket. The caustic solution is added to a charge tank and connected to the feed side of a metering pump.

The reaction mixture is heated to 30 degrees C and the caustic carefully added by means of a

metering pump with stirring to adjust the pH to about 10.9. After about forty minutes at 30 degrees C the addition of the 40% caustic solution is started at a rate of 9.65 milliliters per minute and the temperature of the reaction is increased to 60 degrees C. The addition of caustic required about 45 minutes and the reaction temperature was maintained at 60 degrees C. Gas chromatograph analysis of a sample taken at this time indicated that the reaction was complete and that the *gem*-bis(hydroxymethyl) alcohol corresponding to the formyloctadecanol is formed.

The reaction is held for an additional 20 minutes at 60 degrees C after completion of the caustic addition. The stirring is then stopped and the lower aqueous phase (816 grams) was allowed to separate.

After washing of the crude *gem*-bis(hydroxymethyl) octadecanol and its drying vacuum the amount recovered is 1711 grams corresponding to a yield of greater than 90%.

Alternatively linoleyl or linolenyl alcohol derivatives of Example I may be employed under similar conditions. The reaction temperature for the production of the bishydroxymethyloctadecanol may also be conveniently varied between 0 degrees C and 100 degrees C as previously discussed.

An alternative method of obtaining the bishydroxymethyloctadenol is to use 6.43 moles of the 55.6% formaldehyde solution thereby yielding the corresponding hydroxymethyl formyloctadecanol as an isolatable product. This material is then reduced through catalytic hydrogenation with copper chromite or through the use of lithium aluminum hydride to give the *gem*-bis(hydroxymethyl)octadecanol.

Example III

A polyurethane casting is prepared by reacting 58 parts of the 9,9(10,10)-bis(hydroxymethyl)octadecanol with 59 parts of isophorone-diisocyanate. The mixture is warmed slightly to promote homogeneity. A small portion of dibutyl-tin-dilaurate (.46 parts) is added to the foregoing mixture with rapid stirring whereupon the mixture sets into a clear, colorless, hard solid in about one minute. A distinct advantage in the products of the present invention is that they tend to cure into clear, colorless solids.

Example IV

A one-shot elastomer is prepared by combining 9,9(10,10)-bis(hydroxymethyl)octadecanol and 9(10)hydroxymethyl octadecanol (9:1 ratio) and a 10% sthoichiometric excess of an 80:20 mixture of 2,4-tolylene diisocyanate and 2,6-tolylene diisocyanate for two minutes at room temperature during which time the exothermic reaction raises the temperature to 100 degrees C. At this time the product is poured into a mold and is cured for sixteen hours at 100 degrees C. This product was found to be an extremely hard material having a Shore D hardness of 82 and is a crystal clear solid having a great impact resistance.

Substantially similar results may be obtained in the above reaction by substituting the higher molecular weight *gem*-bis(hydroxymethyl) alcohols previously described at the above proportions with the 9(10)hydroxymethyl octadecanol.

Example V

A polyurethane elastomer of the present invention is produced from a mixture comprising 90% by weight of 9,9(10,10)-bis(hydroxymethyl) octadecanol and 10% by weight of 9(10)hydroxymethyl octadecanol through reaction with a 10% stoichiometric excess of isophorone-diisocyanate for one hour at 80 degrees C followed by pouring the reaction mixture into a mould. The molded specimen is further cured for sixteen hours at 100 degrees C and thereafter removed from the mold. The polyurethane elastomer so obtained is extremely hard having a Shore D hardness of 76 and appears as a crystal clear solid which did not fracture upon striking soundly with a hammer.

Substantially similar results may be obtained in the above reaction by substituting the higher molecular weight *gem*-bis(hydroxymethyl) alcohols previously described at the above proportions with the 9(10)hydroxymethyl octadecanol.

**Claims**

1. A *gem*-bis(hydroxymethyl) alcohol of formula I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[wherein m, n, p, q, r, s and t are integers the sum of which is z which has a value of from 12 to 20; n, q and s each has a value of 0 or 1 with the sum of n, q and s, which is y, having a value of from 1 to 3; m, p and r, which may be the same or different each has a value of from 0 to 16; and t has a value of from 3 to 19].

2. A *gem*-bis(hydroxymethyl alcohol as claimed in claim 1 for which at least one of the following conditions are satisfied (wherein m, n, p, q, r, s, t, y and z are as defined in claim 1):

i) z is from 14 to 18;

ii) y is 1 and z is 16;

iii) y is 2 and z is 16;
iv) y is 3 and z is 16;
v) q is 1 and n and s are zero;
vi) m and t, which may be the same or different, each has a value greater than or equal to 4;
vii) n, p, r and s are zero and q is 1;
viii) t is 7 or 8, s is 1, r is 7 or 8, z is 16 and m, n, p and q are zero;
ix) t is 7 or 8, s is 1, r is 1, 2 or 3, q is 1, p is 4 or 5, z is 16 and m and n are zero; and
x) t is 7 or 8, s is 1, r is 1, 2 or 3, q is 1, p is 1, 2 or 3, n is 1, m is 1 or 2 and z is 16.

3. A mixture of a *gem*-bis (hydroxymethyl alcohol of formula I (as defined in claim 1) and a diol of formula IIA

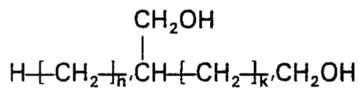

[wherein h' and k' are integers; k' is greater than or equal to 3; h' is greater than or equal to 1; and the sum of h' and k' is from 12 to 20].

4. A mixture as claimed in claim 3 for which at least one of the following conditions is satisfied (wherein h' and k' are as defined in claim 3 and n, p, q, r, s, t and z are as defined in claim 1):
$\alpha$) the sum of h' and k' is from 14 to 18;
$\beta$) the sum of h' and k' is equal to z, h' is greater than or equal to t and k' is greater than or equal to (m+1);
$\gamma$) m, t and h', which may be the same or different, each has a value greater than or equal to 4;
$\delta$) n, p, q, r and s are zero and q is 1;
$\varepsilon$) the diol is 9(10)hydroxymethyl octadecanol;
$\zeta$) the *gem*-bis(hydroxymethyl) alcohol is 9,9(10,10)bis(hydroxymethyl) octadecanol;
$\eta$) the *gem*-bis(hydroxymethyl) alcohol is 9,9(10,10); 12,12(13,13)di[bis(hydroxymethyl)]octadecanol;
$\theta$) the gem-bis(hydroxymethyl) alcohol is 9,9(10,10); 12,12(13,13); 15,15(16,16)tri[bis-(hydroxymethyl)]octadecanol; and
i) the weight ratio of gem alcohol to diol is from about 1:2 to about 100:1.

5. A process for the preparation of a *gem*-bis(hydroxymethyl) alcohol of formula I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[wherein m, n, p, q, r, s and t are integers the sum of which is z which has a value of from 12 to 20; n, q and s each has the value of 0 or 1 with the sum of n, q and s, which is y, having a value of from 1 to 3; m, p, r, which may be the same or different, each has a value of from 0 to 16; and t has a value of from 3 to 19] wherein a formyl alcohol of formula III

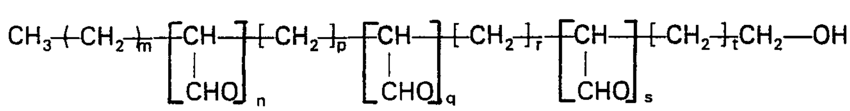
(III)

[wherein m, n, p, q, r, s and t are as herein defined] is subjected to a Tollen's reaction.

6. A process for the preparation of a *gem*-bis(hydroxymethyl) alcohol of formula I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[wherein m, n, p, q, r, s and t are as defined in claim 1] wherein a hydroxymethyl formyl alcohol of formula V

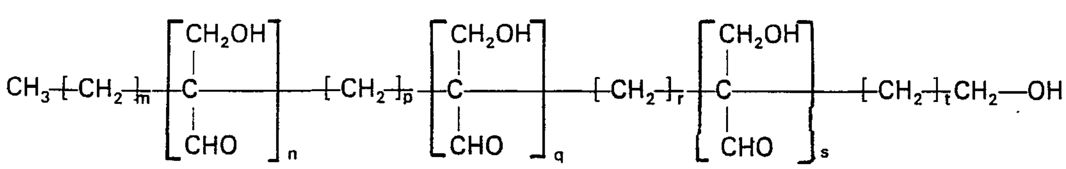
(V)

[wherein m, n, p, q, r, s and t are as defined in claim 1] produced by the subjection of a formyl alcohol of formula III

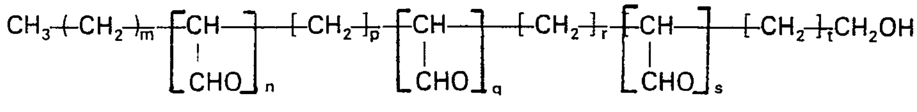
(III)

[wherein m, n, p, q, r, s and t are as herein defined] to an aldol condensation reaction is reduced to produce the said *gem*-bis(hydroxymethyl) alcohol.

7. *Gem*-bis(hydroxymethyl) alcohols of formula I as defined in claim 1 whenever produced by a process as claimed in either of claims 5 and 6.

8. A process for the production of a mixture of a *gem*-bis(hydroxymethyl) alcohol of formula I as defined in claim 1 and a diol of formula II

$$H\left[CH_2\right]_h\overset{\displaystyle CH_2OH}{\overset{|}{C}H}\left[CH_2\right]_k CH_2OH \qquad (II)$$

[wherein h and k are integers; k is greater than or equal to t (wherein t is as hereinafter defined); h is greater than or equal to m+1 (wherein m is as hereinafter defined); and the sum of h and k is equal to z (wherein z is as hereinafter defined)] from a formyl alcohol of formula III

$$CH_3\left[CH_2\right]_m\left[\underset{\displaystyle CHO}{\underset{|}{CH}}\right]_n\left[CH_2\right]_p\left[\underset{\displaystyle CHO}{\underset{|}{CH}}\right]_q\left[CH_2\right]_r\left[\underset{\displaystyle CHO}{\underset{|}{CH}}\right]_s\left[CH_2\right]_t CH_2OH \qquad (III)$$

[wherein m, n, p, q, r, s and t are integers the sum of which is z which has a value of from 12 to 20; n, q and s each has the value of 0 or 1 with the sum of n, q and s, which is y, having a value of from 1 to 3; m, p and r, which may be the same or different, each has a value of from 0 to 16; t has a value of from 3 to 19; h and k are integers, k is greater than or equal to t; h is greater than or equal to m; and the sum of h and k is equal to the sum of m, n, p, q, r, s and t] in which process the said formyl alcohol is split into two parts, one of which is reduced by hydrogenation to produce the said diol, the other being converted to the *gem*-bis(hydroxymethyl) alcohol either by a Tollen's reaction or by an aldol condensation followed by a reduction process, and the diol and gem alcohol so formed subsequently are recombined.

9. A process as claimed in claim 8, wherein the said diol and *gem* alcohol so formed are recombined in a weight ratio of from about 2:1 to about 1:100.

10. Mixtures of *gem*-bis(hydroxymethyl) alcohols and diols of formulae I and II respectively whenever produced by a process as claimed in either of claims 8 and 9.

11. A process for the production of a polyurethane from a *gem*-bis(hydroxymethyl) alcohol of formula I as defined in claim 1 or from a mixture of a *gem*-bis(hydroxymethyl) alcohol and a diol of formula II or IIA as defined in claim 3 or claim 8 by reaction with an organic polyisocyanate.

12. A process as claimed in claim 11, wherein the said mixture of *gem*-bis(hydroxymethyl) alcohol and diol is a mixture as claimed in claim 10.

13. A process as claimed in either of claims 11 and 12, wherein from about 80 to about 120% of the said organic polyisocyanate is used, calculated on a hydroxy-isocyanate equivalent basis (as hereinbefore defined).

14. Polyurethanes whenever produced by a process as claimed in any of claims 11 to 13.

15. Articles whenever incorporating, fabricated from or coated with a polyurethane as claimed in claim 14.

16. A process for the preparation of liquid or solid surfactant products excluding polyurethane which process comprises adducting a *gem*-bis(hydroxymethyl) alcohol as claimed in claim 1 or a mixture of alcohols as claimed in claim 3 with a poly(oxy)alkylene and subsequently sulfating the adducted alcohol or alcohols.

**Patentansprüche**

1. Gem-bis(hydroxymethyl)-alkohol der Formel I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

worin m, n, p, q, r, s und t ganze Zahlen sind, deren Summe z ist, das den Wert von 12 bis 20 besitzt; n, q und s jeweils einen Wert von 0 oder 1 besitzen, wobei die Summe von n, q und s, die y ist, einen Wert von 1 bis 3 besitzt; m, p und r, die gleich oder verschieden sein können, einen Wert von 0 bis 16 besitzen; und t einen Wert von 3 bis 19 besitzt.

2. Gem-bis-(hydroxymethyl)-alkohol nach Anspruch 1, bei dem mindestens eine der folgenden Bedingungen erfüllt ist (worin m, n, p, q, r, s, t, y und z die in Anspruch 1 angegebene Bedeutung besitzen):

i) z 14 bis 18;
ii) y 1 und z 16;
iii) y 2 und z 16;

iv) y 3 und z 16;

v) q 1 und n und s 0 sind;

vi) m und t, die gleich oder verschieden sein können, jeweils einen Wert größer oder gleich 4 aufweisen;

vii) n, p, r und s 0 und q 1;

viii) t 7 oder 8, s 1, r 7 oder 8, z 16 und m, n, p und q 0;

ix) t 7 oder 8, s 1, r 1, 2 oder 3, q 1, p 4 oder 5, z 16 und m und n 0; und

x) t 7 oder 8, s 1, r 1, 2 oder 3, q 1, p 1, 2 oder 3, n 1, m 1 oder 2 und z 16 sind.

3. Gemisch aus einem Gem-bis-(hydroxymethyl)-alkohol der Formel I nach Anspruch 1 und einem Diol der Formel IIA

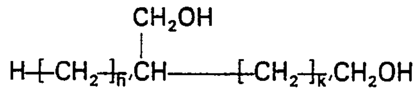
(IIA)

worin h′ und k′ ganze Zahlen, k′ größer als oder gleich 3; h′ größer als oder gleich 1 sind und die Summe von h′ und k′ 12 bis 20 beträgt.

4. Gemisch nach Anspruch 3, bei dem mindestens eine der folgenden Bedingungen erfüllt ist, worin h′ und k′ die in Anspruch 3 angegebene Bedeutung und n, p, q, r, s, t und z die in Anspruch 1 angegebene Bedeutung haben:

α) die Summe von h′ und k′ 14 bis 18 ist;

β) die Summe von h′ und k′ gleich z, h′ größer als oder gleich t und k′ größer als oder gleich (m+1) sind;

γ) m, t und h′, die gleich oder verschieden sein können, einen Wert größer als oder gleich 4 aufweisen;

δ) n, p, q, r und s 0 und q 1 sind;

ε) das Diol 9(10)-Hydroxymethyloctadecanol ist;

ζ) der Gem-bis-(hydroxymethyl)-alkohol 9,9(10,10)-bis-(hydroxymethyl)-octadecanol ist;

η) der Gem-bis-(hydroxymethyl)-alkohol 9,9(10,10); 12,12(13,13)-di-[bis-(hydroxymethyl)]-octadecanol ist;

θ) der Gem-bis-(hydroxymethyl)-alkohol 9,9(10,10); 12,12(13,13); 15,15(16,16)-tri-[bis-(hydroxymethyl)]-octadecanol ist; und

i) das Gewichtsverhältnis des Gem-alkohols zum Diol etwa 1:2 bis etwa 100:1 beträgt.

5. Verfahren zur Herstellung eines Gem-bis-(hydroxymethyl)-alkohols der Formel I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

worin m, n, p, q, r, s und t ganze Zahlen sind, deren Summe z ist, das den Wert von 12 bis 20 besitzt; n, q und s jeweils einen Wert von 0 oder 1 besitzen, wobei die Summe von n, q und s, die y ist, einen Wert von 1 bis 3 besitzt; m, p und r, die gleich oder verschieden sein können, einen Wert von 0 bis 16 besitzen; und t einen Wert von 3 bis 19 besitzt, worin ein Formylalkohol der Formel II

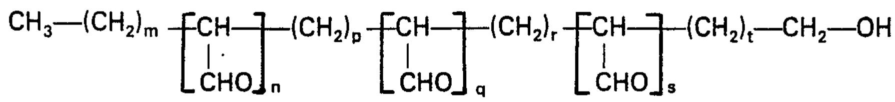
(III)

worin m, n, p, q, r, s und t vorstehende Bedeutung haben, einer Tollens-Reaktion unterworfen wird.

6. Verfahren zur Herstellung eines Gem-bis-(hydroxymethyl)-alkohols der Formel I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

worin m, n, p, q, r, s und t die in Anspruch 1 angegebene Bedeutung haben, worin ein Hydroxymethylformylalkohol der Formel V

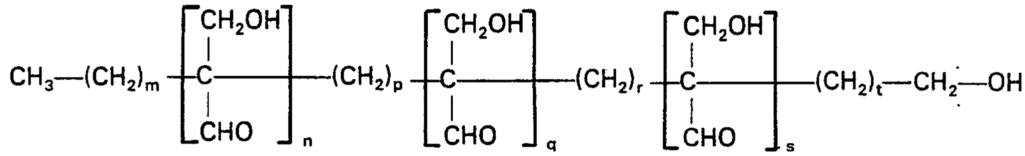
(V)

worin m, n, p, q, r, s und t die in Anspruch 1 angegebene Bedeutung haben, der dadurch erhalten worden ist, daß man einen Formylalkohol der Formel III

$$CH_3-(CH_2)_m-\left[\underset{\displaystyle CHO}{\underset{|}{CH}}\right]_n-(CH_2)_p-\left[\underset{\displaystyle CHO}{\underset{|}{CH}}\right]_q-(CH_2)_r-\left[\underset{\displaystyle CHO}{\underset{|}{CH}}\right]_s-(CH_2)_tCH_2-OH \qquad (III)$$

worin, m, n, p, q, r, s und t vorstehende Bedeutung haben, einer Aldolkondensation unterworfen hat, reduziert wird unter Bildung des Gem-bis-(hydroxymethyl)-alkohols.

7. Gem-bis-(hydroxymethyl)-alkohole der Formel I nach Anspruch 1, hergestellt nach einem Verfahren einer der Ansprüche 5 oder 6.

8. Verfahren zur Herstellung eines Gemischs aus einem Gem-bis-(hydroxymethyl)-alkohol der Formel I nach Anspruch 1, und einem Diol der Formel II

$$H-[CH_2]_h-\overset{\displaystyle CH_2OH}{\overset{|}{CH}}-[CH_2]_k-CH_2OH \qquad (II)$$

worin h und k ganze Zahlen sind, k größer als oder gleich t ist, worin t nachstehende Bedeutung hat; h größer als oder gleich m+1 ist, worin m nachstehende Bedeutung hat; und die Summe von h und k gleich z ist, worin z nachstehende Bedeutung hat, aus einem Formylalkohol der Formel III

$$CH_3-(CH_2)_m-\left[\underset{\displaystyle CHO}{\underset{|}{CH}}\right]_n-(CH_2)_p-\left[\underset{\displaystyle CHO}{\underset{|}{CH}}\right]_q-(CH_2)_r-\left[\underset{\displaystyle CHO}{\underset{|}{CH}}\right]_s-(CH_2)_t-CH_2-OH \qquad (III)$$

worin m, n, p, q, r, s und t ganze Zahlen sind, deren Summe z ist, die einen Wert von 12 bis 20 aufweist; n, q und s jeweils den Wert von 0 oder 1 haben, wobei die Summe von n, q und s, die y ist, einen Wert von 1 bis 3 aufweist; m, p und r, die gleich oder verschieden sein können; jeweils einen Wert von 0 bis 16 besitzen; t einen Wert von 3 bis 19 besitzt; h und k ganze Zahlen sind, wobei k größer als oder gleich t ist; h größer als oder gleich m ist; und die Summe von h und k gleich der Summe von m, n, p, q, r, s und t ist, bei dem der Formylalkohol in zwei Teile gespalten wird, wobei einer davon durch Hydrierung unter Bildung des Diols reduziert wird und der andere in dem Gem-bis-(hydroxymethyl)-alkohol überführt wird, entweder durch eine Tollens-Reaktion oder durch eine Aldol-Kondensation und anschließender Reduktion, und der sich so bildende Diol- und Gem-Alkohol anschließend wieder vereinigt werden.

9. Verfahren nach Anspruch 8, worin der sich bildende Diol- und Gem-Alkohol in einem Gewichtsverhältnis von etwa 2:1 bis etwa 1:100 wieder vereinigt werden.

10. Gemische aus Gem-bis-(hydroxymethyl)-alkoholen und Diolen der Formeln I bzw. II, hergestellt nach einem Verfahren einer der Ansprüche 8 oder 9.

11. Verfahren zur Herstellung eines Polyurethans aus Gem-bis-(hydroxymethyl)-alkohol der Formel I nach Anspruch 1 oder aus einem Gemisch aus einem Gem-bis-(hydroxymethyl)-alkohol und einem Diol der Formel II oder IIA wie in Anspruch 3 oder Anspruch 8 definiert durch Umsetzung mit einem organischen Polyisocyanat.

12. Verfahren nach Anspruch 11, worin das Gemisch aus Gem-bis-(hydroxymethyl)-alkohol und Diol ein Gemisch nach Anspruch 10 ist.

13. Verfahren nach einem der Ansprüche 11 oder 12, worin etwa 80 bis 120% des organischen Polyisocyanats verwendet wird, berechnet auf einer Hydroxy-isocyanat-äquivalenzbasis wie vorstehend definiert.

14. Polyurethane hergestellt nach einem Verfahren einer der Ansprüche 11 bis 13.

15. Gegenstände hergestellt aus oder überzogen mit einem Polyurethane gemäß Anspruch 14.

16. Verfahren zur Herstellung eines flüssigen oder festen oberflächenaktiven Produktes außer Polyurethan, bei dem ein Gem-bis-(hydroxymethyl)-alkohol gemäß Anspruch 1 oder eine Gemisch von Alkoholen gemäß Anspruch 3 mit einem Poly(oxy)alkylen einer Adduktbildung unterworfen wird und anschließend das Addukt von Alkohol oder Alkoholen sulfatiert wird.

**Revendications**

1. Alcool *gem*-bis (hydroxyméthylique) de formule I:

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[dans laquelle m, n, p, q, r, s et t sont des nombres entiers dont la somme z a une valeur de 12 à 20; n, q et s ont chacun une valeur de 0 ou 1, la somme de n, q et s, appelée y, ayant une valeur de 1 à 3; m, p et r, qui peuvent être égaux ou différents ayant chacun une valeur de 0 à 16, et t ayant une valeur de 3 à 19].

2. Alcool *gem*-bis (hydroxyméthylique) suivant la revendication 1, dans lequel l'une au moins des conditions suivantes est satisfaite, (m, n, p, q, r, s, t, y et z ayant les définitions données dans la revendication 1):

i) z a une valeur de 14 à 18;

ii) y est égal à 1 et z est égal à 16;

iii) y est égal à 2 et z est égal à 16;

iv) y est égal à 3 et z est égal à 16;

v) q est égal à 1 et n et s sont égaux à zéro;

vi) m et t, qui peuvent être égaux ou différents ont chacune une valeur supérieure ou égale à 4;

vii) n, p, r et s sont égaux à zéro et q est égal à 1;

viii) t est égal à 7 ou 8, s est égal à 1, r est égal à 7 ou 8, z est égal à 16; et m, n, p et q sont égaux à zéro;

ix) t est égal à 7 ou 8, s est égal à 1, r est égal à 1, 2 ou 3, q est égal à 1, p est égal à 4 ou 5, z est égal à 16 et m et n sont égaux à zéro; et

x) t est égal à 7 ou 8, s est égal à 1, r est égal à 1, 2 ou 3, q est égal à 1, p est égal à 1, 2 ou 3, n est égal à 1, m est égal à 1 ou 2 et z est égal à 16.

3. Un mélange d'un alcool *gem*-bis (hydroxyméthylique) de formule I (suivant la revendication 1) et d'un diol de formule IIA

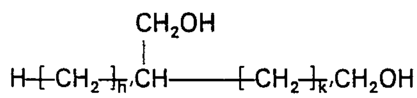

[dans laquelle h' et k' sont des nombres entiers; k' est supérieur ou égal à 3; h' est supérieur ou égal à 1; et la somme de h' et k' van de 12 à 20].

4. Mélange suivant la revendication 3, dans lequel l'une au moins des conditions suivantes est satisfaite (h' et k' ayant les définitions données dans la revendication 3 et n , p, q, r, s, t et z ayant les définitions données dans la revendication 1):

$\alpha$) la somme de h' et k' va de 14 à 18;

$\beta$) la somme de h' et k' est égale à z, h' est supérieur ou égal à t et k' est supérieur ou égal à (m+1);

$\gamma$) m, t et h', qui peuvent être égaux ou différents, ont chacun une valeur supérieure ou égale à 4;

$\delta$) n, p, q, r et s sont égaux à zéro et q est égal à 1;

$\varepsilon$) le diol est le 9 (10) hydroxyméthyloctadécanol;

$\zeta$) l'alcool *gem*-bis (hydroxyméthylique) est le 9,9(10,10)bis(hydroxyméthyl)octadécanol;

$\eta$) l'alcool *gem*-bis (hydroxyméthylique) est le 9,9(10,10); 12,12(13,13)di[bis(hydroxyméthyl)]-octadécanol;

$\theta$) l'alcool gem-bis (hydroxyméthylique) est le 9,9(10,10); 12,12(13,13); 15,15(16,16)tri[bis-(hydroxyméthyl)]octadécanol; et

i) le rapport en poids de l'alcool géminé au diol va d'environ 1:2 à environ 100:1.

5. Procédé de préparation d'un alcool *gem*-bis (hydroxyméthylique) de formule I:

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[dans laquelle m, n, p, q, r, s et t sont des nombres entiers dont la somme z a une valeur de 12 à 20; n, q et s ont chacun une valeur de 0 ou 1, la somme de n, q et s, appelée y, ayant une valeur de 1 à 3; m, p, et r, qui peuvent être égaux ou différents, ayant chacun une valeur de 0 à 16; et t ayant une valeur de 3 à 19], dans lequel un alcool formylique de formule III:

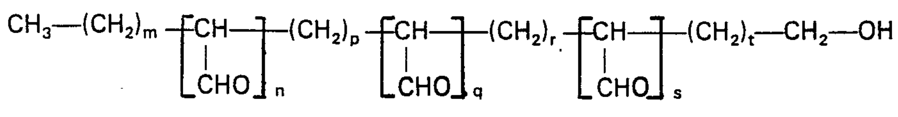
(III)

[où m, n, p, q, r, s et t ont les définitions données ci-dessus] est soumis à une réaction de Tollen.

6. Procédé de préparation d'un alcool *gem*-bis (hydroxyméthylique) de formule I

$$CH_3(CH_2)_m(C(CH_2OH)_2)_n(CH_2)_p(C(CH_2OH)_2)_q(CH_2)_r(C(CH_2OH)_2)_s(CH_2)_tCH_2OH \qquad (I)$$

[où m, n, p, q, r, s et t ont les définitions données dans la revendication 1] dans lequel un alcool hydroxyméthylformylique de formule V:

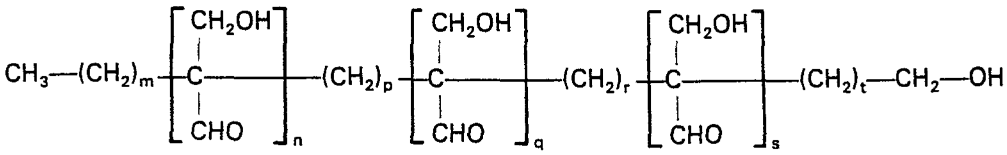 (V)

[dans laquelle, m, n, p, q, r, s et t ont les définitions données dans la revendication 1] produit en soumettant un alcool formylique de formule III:

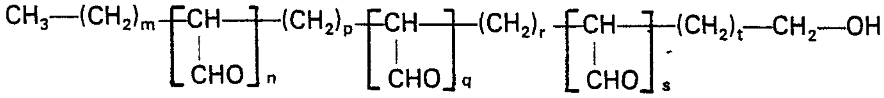 (III)

[où m, n, p, q, r, s et t ont les définitions données ci-dessus] à une réaction de condensation en aldol est réduit pour produire ledit alcool *gem*-bis (hydroxyméthylique).

7. Alcools *gem*-bis (hydroxyméthyliques) de formule I suivant la revendication 1, obtenus par un procédé suivant l'une des revendications 5 et 6.

8. Procédé de production d'un mélange d'un alcool *gem*-bis (hydroxyméthylique) de formule I comme défini dans la revendication 1 et d'un diol de formule II

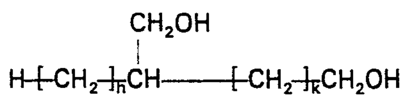 (II)

[où h et k sont des nombres entiers; k est supérieur ou égal à t (t ayant la définition donnée ci-après); h est supérieur ou égal à m+1 (m ayant la définition donnée ci-après); et la somme de h et k est égale à z (z ayant la définition donnée ci-après)] à partir d'un alcool formylique de formule III:

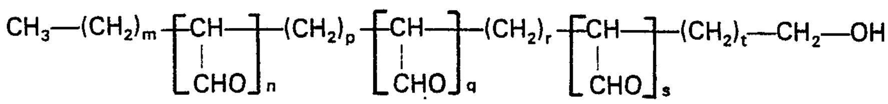 (III)

[où m, n, p, q, r, s et t sont des nombres entiers dont la somme z a une valeur de 12 à 20; n, q et s ont la valeur zéro ou 1, la somme y de n, q et s ayant une valeur de 1 à 3; m, p et r, qui peuvent être égaux ou différents, ont chacun une valeur de 0 à 16; t a une valeur de 3 à 19; h et k sont des nombres entiers, k est supérieur ou égal à t; h est supérieur ou égal à m; et la somme de h et k est égale à la somme de m, n, p, q, r, s et t], procédé dans lequel ledit alcool formylique est scindé en deux parties dont l'une est réduite par hydrogénation pour produire ledit diol, l'autre étant converti en l'alcool *gem*-bis (hydroxyméthylique) soit par une réaction de Tollen, soit par une condensation en aldol suivie d'un processus de réduction, et le diol et l'alcool géminé ainsi formés sont ensuite recombinés.

9. Procédé suivant la revendication 8, dans lequel le diol et l'alcool géminé ainsi formés sont recombinés dans un rapport en poids d'environ 2:1 à environ 1:100.

10. Des mélanges d'alcools *gem*-bis (hydroxyméthyliques) et de diols de formules respectivement I et II obtenus par un prodédé suivant l'une des revendications 8 et 9.

11. Procédé de production d'un polyuréthane à partir d'un alcool *gem*-bis (hydroxyméthylique) de formule I suivant la revendication 1 ou à partir d'un mélange d'un alcool *gem*-bis (hydroxyméthylique) et d'un diol de formule II ou IIA comme défini dans la revendication 3 ou dans la revendication 8, par réaction avec un polyisocyanate organique.

12. Procédé suivant la revendication 11, dans lequel ledit mélange d'alcool *gem*-bis (hydroxyméthylique) et de diol est un mélange suivant la revendication 10.

13. Procédé suivant l'une des revendications 11 et 12, dans lequel on utilise environ 80 à environ 120% dudit polyisocyanate organique, la proportion étant calculée sur une base d'équivalents d'hydroxyisocyanate (comme défini dans ce qui précède).

14. Des polyuréthannes obtenus par un procédé suivant l'une quelconque des revendications 11 à 13.

15. Des articles renfermant un polyuréthanne, obtenus à partir d'un polyuréthanne ou revêtus d'un polyuréthanne suivant la revendication 14.

16. Procédé de préparation de produits surfactants liquides ou solides à l'exclusion de polyuréthanne, ce procédé consistant à additionner un alcool *gem*-bis (hydroxyméthylique) suivant la revendication 1 ou un mélange d'alcools suivant la revendication 3 avec un poly(oxy)alkylène et à former ensuite l'ester sulfurique de l'alcool ou des alcools additionnés.